# EUROPEAN PATENT SPECIFICATION

(11) **EP 1 097 999 B1**
(45) Date of publication and mention of the grant of the patent: **18.08.2004**
(21) Application number: 00305459.0
(22) Date of filing: 29.06.2000
(51) Int. Cl.: C12N 15/82, C12N 15/29, C07K 14/415

(54) **Abscisic acid responsive element-binding transcription factors**
An das durch Abscisinsäure-aktivierbare-Element bindende Transkriptionsfaktoren
Facteurs de transcription se liant à l'élèment sensible à l'acide abscisique

(30) Priority: 04.11.1999 KR 9948477
(43) Date of publication of application: 09.05.2001
(73) Proprietor: Korea Kumho Petrochemical Co. Ltd., Seoul (KR)
(72) Inventor: Kim, Soo Young, 1 Oryong-Dong, Puk-Gu, Kwangju 500-712 (KR)
(74) Representative: Harding, Charles Thomas

(56) References cited:
- KIM SOO YOUNG ET AL: "Isolation of a novel class of bZIP transcription factors that interact with ABA-responsive and embryo-specification elements in the Dc3 promoter using a modified yeast one-hybrid system." PLANT JOURNAL, vol. 11, no. 6, 1997, pages 1237-1251, XP002208277 ISSN: 0960-7412
- DATABASE EMBL 7 July 1998 (1998-07-07) "Arabidopsis thaliana DNA chromosome 4, BAC clone F17I5 (ESSA project)" Database accession no. AL031032 XP002208292
- KASUGA MIE ET AL: "Improving plant drought, salt, and freezing tolerance by gene transfer of a single stress-inducible transcription factor" NATURE BIOTECHNOLOGY, NATURE PUB. CO, NEW YORK, NY, US, vol. 17, no. 3, March 1999 (1999-03), pages 287-291, XP002173128 ISSN: 1087-0156
- KIM SOO YOUNG ET AL: "A family of novel basic leucine zipper proteins binds to seed-specification elements in the carrot Dc3 gene promoter." JOURNAL OF PLANT PHYSIOLOGY, vol. 152, no. 6, June 1998 (1998-06), pages 607-613, XP008006363 ISSN: 0176-1617
- BUSK PETER K ET AL: "Regulation of abscisic acid-induced transcription" PLANT MOLECULAR BIOLOGY, NIJHOFF PUBLISHERS, DORDRECHT, NL, vol. 37, no. 3, June 1998 (1998-06), pages 425-435, XP002185939 ISSN: 0167-4412
- CHOI HYUNG-IN ET AL: "ABFs, a family of ABA-responsive element binding factors." JOURNAL OF BIOLOGICAL CHEMISTRY, vol. 275, no. 3, 21 January 2000 (2000-01-21), pages 1723-1730, XP002204935 ISSN: 0021-9258

### SEQUENCE LISTING

<110> KOREA KUMHO PETROCHEMICAL Co., LTD.
<120> Abscisic acid responsive element―binding transcription factors
<130>
<160> 8
<210> 1
<211> 1578
<212> DNA
<213> Arabidopsis sp.
<220>
<223> single strand
<223> topology, linear
<223> molecular type, cDNA to mRNA
<400> 1
   AAAGGGTCTG ATTCGTTTGT TTTTTCACTG AAGAATTTGG AAGGAAGTGA TTCCGTTGTG    60
   AAACAGAAAA GAAGTATGGG TACTCACATT GATATCAACA ACTTAGGCGG CGATACTTCT   120
   AGAGGGAATG AGTCAAAGCC ATTGGCGAGG CAGTCTTCGT TATATTCCTT AACGTTTGAT   180
   GAGCTTCAGA GCACATTAGG TGAGCCGGGG AAAGATTTTG GGTCTATGAA TATGGATGAG   240
   TTACTCAAGA ACATATGGAC TGCTGAGGAT ACTCAAGCCT TTATGACTAC TACATCTTCG   300
   GTTGCAGCCC CGGGACCTAG TGGTTTTGTT CCGGGAGGAA ATGGTTTACA GAGGCAAGGC   360
   TCCTTGACCT TGCCTAGAAC GCTTAGTCAG AAGACTGTCG ATGAAGTCTG GAAATACCTG   420
   AATTCGAAAG AAGGTAGTAA TGGGAATACT GGAACGGATG CGCTTGAGAG GCAACAGACT   480
   TTAGGGGAAA TGACTCTGGA AGATTTCTTA CTCCGTGCTG GCGTTGTTAA AGAAGATAAT   540
   ACTCAGCAGA ACGAAAACAG TAGTAGCGGG TTTTATGCTA ACAACGGTGC TGCTGGTTTG   600
   GAGTTTGGAT TTGGTCAGCC GAATCAAAAC AGCATATCGT TCAACGGGAA CAATAGTTCT   660
   ATGATCATGA ATCAAGCACC TGGTTTAGGC CTCAAAGTTG GTGGAACCAT GCAGCAGCAG   720
   CAGCAGCCAC ATCAGCAGCA GTTGCAGCAG CCACATCAGA GACTGCCTCC AACTATCTTT   780
   CCAAAACAAG CGAATGTAAC ATTTGCGGCG CCTGTAAATA TGGTCAACAG GGGTTTATTT   840
   GAGACTAGCG CAGATGGTCC AGCCAACAGT AATATGGGAG GAGCAGGGGG TACTGTTACA   900
   GCTACTTCTC CTGGGACGAG CAGTGCAGAA AACAATACTT GGTCATCACC AGTTCCTTAC   960
   GTGTTTGGTC GGGGAAGAAG AAGCAATACG GGCCTGGAGA AGGTTGTTGA GAGAAGGCAA  1020
   AAGAGAATGA TCAAGAATCG GGAATCCGCT GCTAGATCAA GGGCTCGAAA ACAGGCTTAT  1080
   ACCTTGGAAC TGGAAGCTGA GATTGAAAGT CTCAAGCTAG TGAATCAAGA TTTGCAGAAG  1140
   AAACAGGCTG AAATAATGAA AACCCATAAT AGTGAGCTAA AGGAATTTTC GAAGCAGCCT  1200
   CCATTGCTGG CCAAAAGACA ATGCTTGAGA AGAACCCTTA CCGGTCCGTG GTAAGAAGGT  1260
   GAAGTCAAAG CAAGAAGAAC CTGCTAATGT AATACAGGAC CACTCAAAAG GAAGACACTG  1320
   GGAGAGTAAT ATGTAATAGA AGATAGTGCT ACTGTACAGG AGAAATTACA GAGACGCTTA  1380
   CAATGTAGAA ATCTTTTGAG CTGAATTTAA CTAAGAGTGC AGTCTGTGTA GAGTATGAGA  1440
   GCTTTCAATA TGAATTCATA ATTTTCATAA ACATATGTAA AACTTTCAGA TTTAGCTATA  1500
   GAGAAGATGT GACTAAAAAA AAAAAAAAAA AAAAAAAAAA AAAAAAAAAA AAAAAAAAAA  1560
   AAAAAAAAAA AAAAAAAA                                                1578
<210> 2
<211> 392
<212> amino acid
<213> Arabidopsis sp.
<220>
<223>
<223> topology, linear
<223> molecular type, deduced amino acid sequence of SEQ ID NO : 1
<400> 2
<210> 3
<211> 1654
<212> DNA
<213> arabidopsis sp.
<220>
<223> single strand
<223> topology, linear
<223> molecular type, cDNA to mRNA
<400> 3
   CCCAAACGAA GAACCAAACA TTTTGAAATT TTTTGGGAAA ATTACAAAGC ACACGAATTT    60
   AGCAAAAAGA TCCAGTTATT AGGTGGAAGC AGATTTTGTA GAAAAATGGA TGGTAGTATG   120
   AATTTGGGGA ATGAGCCACC AGGAGATGGT GGTGGAGGTG GAGGGTTGAC TAGACAAGGT   180
   TCGATATACT CGTTGACGTT TGATGAGTTT CAGAGCAGTG TAGGGAAAGA TTTTGGGTCA   240
   ATGAACATGG ATGAGTTGTT AAAGAATATA TGGAGTGCTG AAGAAACACA AGCCATGGCT   300
   AGTGGTGTGG TTCCAGTTCT TGGTGGAGGT CAAGAGGGTT TGCAGCTGCA GAGGCAAGGC   360
   TCGTTGACTC TGCCTCGAAC GCTTAGTCAG AAGACGGTTG ATCAAGTTTG GAAAGATCTA   420
   TCCAAAGTTG GAAGTAGTGG AGTAGGGGGA AGTAACTTGT CTCAGGTGGC TCAGGCTCAG   480
   AGTCAGAGTC AGAGTCAGAG GCAGCAAACA TTAGGTGAAG TAACTTTGGA GGAGTTTTTG   540
   GTTCGTGCTG GTGTTGTGAG AGAGGAAGCT CAGGTTGCTG CAAGAGCTCA GATTGCTGAG   600
   AACAATAAAG GCGGTTACTT TGGTAATGAT GCCAACACAG GTTTCTCTGT CGAGTTTCAG   660
   CAGCCTTCTC CACGAGTTGT TGCCGCTGGT GTAATGGGAA ATCTTGGTGC AGAGACTGCA   720
   AATTCTTTGC AGGTTCAAGG TTCTAGTTTG CCTCTGAATG TGAATGGAGC TAGAACAACA   780
   TACCAGCAAT CGCAACAGCA ACAGCCAATC ATGCCTAAGC AGCCTGGTTT TGGTTATGGA   840
   ACACAAATGG GTCAGCTTAA TAGTCCTGGG ATAAGAGGTG GTGGTCTTGT GGGACTTGGA   900
   GATCAGTCTT TAACGAACAA TGTGGGCTTT GTCCAAGGTG CTTCTGCTGC AATTCCTGGA   960
   GCTTTAGGCG TTGGTGCTGT GTCGCCTGTT ACGCCATTGT CATCAGAAGG GATAGGGAAG  1020
   AGTAATGGTG ATTCTTCATC ACTCTCTCCG TCTCCTTACA TGTTTAATGG TGGTGTGAGA  1080
   GGTAGAAAGA GTGGCACTGT GGAGAAAGTT GTAGAGAGAA GGCAAAGGAG AATGATAAAG  1140
   AACCGAGAAT CAGCTGCAAG GTCCCGGGCC AGGAAACAGG CTTACACCGT GGAGCTTGAA  1200
   GCTGAAGTTG CAAAGTTAAA GGAAGAGAAT GACGAGTTAC AACGAAAGCA GGCAAGGATC  1260
   ATGGAAATGC AAAAGAATCA GGAGACGGAG ATGAGGAATC TTCTGCAAGG AGGTCCAAAG  1320
   AAAAAGCTGA GGAGGACAGA GTCGGGACCT TGGTGAATCA ATCAATGCCA TCATACTTAG  1380
   TTTCTGTAGA TAAATGACAT CCCACTTAGG TGTTTTAGTT GAATTAGACT TAATAGAGAA  1440
   GAGCTTTCAT CGTTTATATT GTAAGCTCTC TCCATATATG TTATGTTTTT TACATACACA  1500
   GGATCATCAG AATCTCTTTT GCTTTATTTA GACCAAGAAT TTTGTGTGTG TTTCTCGTTG  1560
   TTGTTTGTCG TTGTCGCTAT TAAACCTCAA AATGTACTTT CTTGATCTTG GAGTTACCAA  1620
   TTTTGAAGAA TTGAAGTGTT GTTTGGTTAA AAAA                              1654
<210> 4
<211> 416
<212> amino acid
<213> Arabidopsis sp.
<220>
<223>
<223> topology, linear
<223> molecular type, deduced amino acid sequence of SEQ ID NO : 3
<400> 4
   Met Asp Gly Ser Met Asn Leu Gly Asn Glu Pro Pro Gly Asp Gly Gly Gly
   1               5                   10                  15
   Gly Gly Gly Leu Thr Arg Gln Gly Ser Ile Tyr Ser Leu Thr Phe Asp Glu
           20                  25                  30
   Phe Gln Ser Ser Val Gly Lys Asp Phe Gly Ser Met Asn Met Asp Glu Leu
   35                  40                  45                      50
   Leu Lys Asn Ile Trp Ser Ala Glu Glu Thr Gln Ala Met Ala Ser Gly Val
               55                  60                  65
   Val Pro Val Leu Gly Gly Gly Gln Glu Gly Leu Gln Leu Gln Arg Gln Gly
       70                  75                  80                  85
   Ser Leu Thr Leu Pro Arg Thr Leu Ser Gln Lys Thr Val Asp Gln Val Trp
                   90                  95                  100
   Lys Asp Leu Ser Lys Val Gly Ser Ser Gly Val Gly Gly Ser Asn Leu Ser
           105                 110                 115
   Gln Val Ala Gln Ala Gln Ser Gln Ser Gln Ser Gln Arg Gln Gln Thr Leu
   120                 125                 130                 135
   Gly Glu Val Thr Leu Glu Glu Phe Leu Val Arg Ala Gly Val Val Arg Glu
               140                 145                 150
   Glu Ala Gln Val Ala Ala Arg Ala Gln Ile Ala Glu Asn Asn Lys Gly Gly
       155                 160                 165                 170
   Tyr Phe Gly Asn Asp Ala Asn Thr Gly Phe Ser Val Glu Phe Gln Gln Pro
                   175                 180                 185
   Ser Pro Arg Val Val Ala Ala Gly Val Met Gly Asn Leu Gly Ala Glu Thr
           190                 195                 200
   Ala Asn Ser Leu Gln Val Gln Gly Ser Ser Leu Pro Leu Asn Val Asn Gly
   205                 210                 215                 220
   Ala Arg Thr Thr Tyr Gln Gln Ser Gln Gln Gln Gln Pro Ile Met Pro Lys
               225                 230                 235
   Gln Pro Gly Phe Gly Tyr Gly Thr Gln Met Gly Gln Leu Asn Ser Pro Gly
       240                 245                 250                 255
   Ile Arg Gly Gly Gly Leu Val Gly Leu Gly Asp Gln Ser Leu Thr Asn Asn
                   260                 265                 270
   Val Gly Phe Val Gln Gly Ala Ser Ala Ala Ile Pro Gly Ala Leu Gly Val
           275                 280                 285
   Gly Ala Val Ser Pro Val Thr Pro Leu Ser Ser Glu Gly Ile Gly Lys Ser
   290                 295                 300                 305
   Asn Gly Asp Ser Ser Ser Leu Ser Pro Ser Pro Tyr Met Phe Asn Gly Gly
               310                 315                 320
   Val Arg Gly Arg Lys Ser Gly Thr Val Glu Lys Val Val Glu Arg Arg Gln
       325                 330                 335                 340
   Arg Arg Met Ile Lys Asn Arg Glu Ser Ala Ala Arg Ser Arg Ala Arg Lys
                   345                 350                 355
   Gln Ala Tyr Thr Val Glu Leu Glu Ala Glu Val Ala Lys Leu Lys Glu Glu
           360                 365                 370
   Asn Asp Glu Leu Gln Arg Lys Gln Ala Arg Ile Met Glu Met Gln Lys Asn
   375                 380                 385                 390
   Gln Glu Thr Glu Met Arg Asn Leu Leu Gln Gly Gly Pro Lys Lys Lys Leu
               395                 400                 405
   Arg Arg Thr Glu Ser Gly Pro Trp
       410                 415
<210> 5
<211> 1685
<212> DNA
<213> arabidopsis sp.
<220>
<223> single strand
<223> topology, linear
<223> molecular type, cDNA to mRNA
<400> 5
   GAAGCTTGAT CCTCCTAGTT GTACGAAAGC TTGAGTAATG GGGTCTAGAT TAAACTTCAA     60
   GAGCTTTGTT GATGGTGTGA GTGAGCAGCA GCCAACGGTG GGGACTAGTC TTCCATTGAC    120
   TAGGCAGAAC TCTGTGTTCT CGTTAACCTT TGATGAGTTT CAGAACTCAT GGGGTGGTGG    180
   AATTGGGAAA GATTTTGGGT CTATGAACAT GGATGAGCTC TTGAAGAACA TTTGGACTGC    240
   AGAGGAAAGT CATTCAATGA TGGGAAACAA TACCAGTTAC ACCAACATCA GCAATGGTAA    300
   TAGTGGAAAC ACTGTTATTA ACGGCGGTGG TAACAACATT GGTGGGTTAG CTGTTGGTGT    360
   GGGAGGAGAA AGTGGTGGTT TTTTCACTGG TGGGAGTTTG CAGAGACAAG GTTCACTTAC    420
   CTTGCCTCGG ACGATTAGTC AGAAAAGGGT TGATGATGTC TGGAAGGAGC TGATGAAGGA    480
   GGATGACATT GGAAATGGTG TTGTTAATGG TGGGACAAGC GGAATTCCGC AGAGGCAACA    540
   AACGCTGGGA GAGATGACTT TGGAGGAGTT TTTGGTCAGG GCTGGTGTGG TTAGGGAAGA    600
   ACCTCAACCG GTGGAGAGTG TAACTAACTT CAATGGCGGA TTCTATGGAT TTGGCAGTAA    660
   TGGAGGTCTT GGGACAGCTA GTAATGGGTT TGTTGCAAAC CAACCTCAAG ATTTGTCAGG    720
   AAATGGAGTA GCGGTGAGAC AGGATCTGCT GACTGCTCAA ACTCAGCCAC TACAGATGCA    780
   GCAGCCACAG ATGGTGCAGC AGCCACAGAT GGTGCAGCAG CCGCAACAAC TGATACAGAC    840
   GCAGGAGAGG CCTTTTCCCA AACAGACCAC TATAGCATTT TCCAACACTG TTGATGTGGT    900
   TAACCGTTCT CAACCTGCAA CACAGTGCCA GGAAGTGAAG CCTTCAATAC TTGGAATTCA    960
   TAACCATCCT ATGAACAACA ATCTACTGCA AGCTGTCGAT TTTAAAACAG GAGTAACGGT   1020
   TGCAGCAGTA TCTCCTGGAA GCCAGATGTC ACCTGATCTG ACTCCAAAGA GCGCCCTGGA   1080
   TGCATCTTTG TCCCCTGTTC CTTACATGTT TGGGCGAGTG AGAAAAACAG GTGCAGTTCT   1140
   GGAGAAAGTG ATTGAGAGAA GGCAAAAAAG GATGATAAAG AATAGGGAAT CAGCTGCAAG   1200
   ATCCCGCGCT CGCAAGCAAG CTTATACGAT GGAACTGGAA GCAGAAATTG CGCAACTCAA   1260
   AGAATTGAAT GAAGAGTTGC AGAAGAAACA AGTTGAAATC ATGGAAAAGC AGAAAAATCA   1320
   GCTTCTGGAG CCTCTGCGCC AGCCATGGGG AATGGGATGC AAAAGGCAAT GCTTGCGAAG   1380
   GACATTGACG GGTCCCTGGT AGAGCTTATA ATGGCGTCTA AGGAACCCAA CAAAGCGCCG   1440
   AAGTTATAGA ACAACTCAGA AGATAGAAAG CTAGCTTTGT ACGTAGTTTA GGCAGGTTCT   1500
   GTGGGTGATT GTAAATCTTG AAGTGTGGCG GATTTGACAG AGATAGATAA ACACATATCT   1560
   GTTCTATTTT CCTAAATCTT TTGGTTTTAT CTTCCTGATG TAATGGATCT TTATCATTTG   1620
   TCTTGAACAT CTTTGTGACT TAACCAGAGT GAATTTATCT TGTATCTAAA AAAAAAAAAA   1680
   AAAAA                                                               1685
<210> 6
<211> 454
<212> amino acid
<213> Arabidopsis sp.
<220>
<223>
<223> topology, linear
<223> molecular type, deduced amino acid sequence of SEQ ID NO : 5
<400> 6
   Met Gly Ser Arg Leu Asn Phe Lys Ser Phe Val Asp Gly Val Ser Glu Gln
    1              5                   10                  15
   Gln Pro Thr Val Gly Thr Ser Leu Pro Leu Thr Arg Gln Asn Ser Val Phe
           20                  25                  30
   Ser Leu Thr Phe Asp Glu Phe Gln Asn Ser Trp Gly Gly Gly Ile Gly Lys
   35                  40                  45                      50
   Asp Phe Gly Ser Met Asn Met Asp Glu Leu Leu Lys Asn Ile Trp Thr Ala
               55                  60                  65
   Glu Glu Ser His Ser Met Met Gly Asn Asn Thr Ser Tyr Thr Asn Ile Ser
       70                  75                  80                      85
   Asn Gly Asn Ser Gly Asn Thr Val Ile Asn Gly Gly Gly Asn Asn Ile Gly
                   90                  95                  100
   Gly Leu Ala Val Gly Val Gly Gly Glu Ser Gly Gly Phe Phe Thr Gly Gly
           105                 110                 115
   Ser Leu Gln Arg Gln Gly Ser Leu Thr Leu Pro Arg Thr Ile Ser Gln Lys
   120                 125                 130                 135
   Arg Val Asp Asp Val Trp Lys Glu Leu Met Lys Glu Asp Asp Ile Gly Asn
               140                 145                 150
   Gly Val Val Asn Gly Gly Thr Ser Gly Ile Pro Gln Arg Gln Gln Thr Leu
       155                 160                 165                     170
   Gly Glu Met Thr Leu Glu Glu Phe Leu Val Arg Ala Gly Val Val Arg Glu
                   175                 180                 185
   Glu Pro Gln Pro Val Glu Ser Val Thr Asn Phe Asn Gly Gly Phe Tyr Gly
           190                 195                 200
   Phe Gly Ser Asn Gly Gly Leu Gly Thr Ala Ser Asn Gly Phe Val Ala Asn
   205                 210                 215                 220
   Gln Pro Gln Asp Leu Ser Gly Asn Gly Val Ala Val Arg Gln Asp Leu Leu
               225                 230                 235
   Thr Ala Gln Thr Gln Pro Leu Gln Met Gln Gln Pro Gln Met Val Gln Gln
       240                 245                 250                 255
   Pro Gln Met Val Gln Gln Pro Gln Gln Leu Ile Gln Thr Gln Glu Arg Pro
                   260                 265                 270
   Phe Pro Lys Gln Thr Thr Ile Ala Phe Ser Asn Thr Val Asp Val Val Asn
           275                 280                 285
   Arg Ser Gln Pro Ala Thr Gln Cys Gln Glu Val Lys Pro Ser Ile Leu Gly
   290                 295                 300                 305
   Ile His Asn His Pro Met Asn Asn Asn Leu Leu Gln Ala Val Asp Phe Lys
               310                 315                 320
   Thr Gly Val Thr Val Ala Ala Val Ser Pro Gly Ser Gln Met Ser Pro Asp
       325                 330                 335                 340
   Leu Thr Pro Lys Ser Ala Leu Asp Ala Ser Leu Ser Pro Val Pro Tyr Met
                   345                 350                 355
   Phe Gly Arg Val Arg Lys Thr Gly Ala Val Leu Glu Lys Val Ile Glu Arg
           360                 365                 370
   Arg Gln Lys Arg Met Ile Lys Asn Arg Glu Ser Ala Ala Arg Ser Arg Ala
   375                 380                 385                 390
   Arg Lys Gln Ala Tyr Thr Met Glu Leu Glu Ala Glu Ile Ala Gln Leu Lys
               395                 400                 405
   Glu Leu Asn Glu Glu Leu Gln Lys Lys Gln Val Glu Ile Met Glu Lys Gln
       410                 415                 420                 425
   Lys Asn Gln Leu Leu Glu Pro Leu Arg Gln Pro Trp Gly Met Gly Cys Lys
                   430                 435                 440
   Arg Gln Cys Leu Arg Arg Thr Leu Thr Gly Pro Trp
           445                 450
<210> 7
<211> 1737
<212> DNA
<213> arabidopsis sp.
<220>
<223> single strand
<223> topology, linear
<223> molecular type, cDNA to mRNA
<400> 7
   GAACAAGGGT TTTAGGGCTT GGATGCTTTG TTTTCATTGA AAAAGAAGTA GAAGGAGTGT    60
   ATACAAGGAT TATGGGAACT CACATCAATT TCAACAACTT AGGAGGTGGT GGTCATCCTG   120
   GAGGGGAAGG GAGTAGTAAC CAGATGAAGC CAACGGGTAG TGTCATGCCC TTGGCTAGGC   180
   AGTCCTCGGT CTACTCCCTT ACCTTTGATG AGTTACAGAA CACACTAGGT GGACCGGGAA   240
   AAGATTTCGG GTCGATGAAC ATGGATGAAC TCCTGAAGAG CATATGGACT GCTGAGGAAG   300
   CTCAGGCCAT GGCCATGACT TCTGCGCCAG CTGCTACAGC GGTAGCGCAA CCTGGTGCTG   360
   GTATCCCACC CCCAGGTGGG AATCTCCAGA GGCAAGGTTC GTTGACGTTG CCTAGAACAA   420
   TTAGTCAGAA GACTGTTGAT GAGGTGTGGA AATGTTTGAT CACCAAGGAT GGTAATATGG   480
   AAGGTAGCAG CGGAGGCGGT GGTGAGTCGA ATGTGCCTCC TGGAAGGCAA CAGACTTTAG   540
   GGGAAATGAC ACTTGAAGAA TTTCTGTTCC GTGCTGGGGT TGTAAGAGAA GATAACTGTG   600
   TTCAACAGAT GGGTCAGGTC AACGGAAACA ATAACAATGG GTTTTATGGT AACAGCACTG   660
   CTGCTGGCGG CTTAGGTTTT GGATTTGGTC AGCCAAATCA AAACAGCATA ACATTCAATG   720
   GTACTAATGA TTCTATGATC TTGAATCAGC CACCTGGTTT AGGGCTCAAA ATGGGTGGAA   780
   CAATGCAGCA GCAACAACAA CAACAGCAGT TGCTTCAGCA GCAACAACAG CAGATGCAGC   840
   AGCTGAATCA GCCTCATCCA CAGCAGCGGC TGCCTCAAAC CATTTTTCCT AAACAAGCAA   900
   ACGTAGCATT TTCTGCGCCT GTGAATATAA CCAACAAGGG TTTTGCTGGG GCTGCAAATA   960
   ATTCTATCAA CAATAATAAT GGATTAGCTA GTTACGGAGG AACCGGGGTC ACTGTTGCAG  1020
   CAACTTCTCC AGGAACAAGC AGCGCAGAAA ATAATTCTTT ATCACCAGTT CCGTATGTGC  1080
   TTAATCGAGG ACGAAGAAGC AATACAGGTC TAGAGAAGGT TATCGAGAGG AGGCAAAGGA  1140
   GAATGATCAA GAATCGGGAA TCAGCTGCTA GATCAAGAGC TCGAAAGCAG GCTTATACAT  1200
   TGGAACTGGA AGCCGAAATT GAAAAGCTCA AGAAAACGAA TCAAGAACTG CAGAAAAAAC  1260
   AGGCTGAAAT GGTGGAAATG CAGAAGAATG AGCTGAAAGA AACGTCGAAG CGACCGTGGG  1320
   GCAGCAAAAG GCAATGCTTG AGAAGGACAT TAACCGGACC ATGGTGAAGG ATGAAGCAAC  1380
   AAGAACGGAT GAACCAGACT CCTAGCTTGG GATTAATGTA ATAGGATAGT GCTACCTGTA  1440
   CAGGAGATTA AGAGAAATTG AGTGAAAGAT CTAGGTTACA GAGTAGGAGA GAGTTTTCAT  1500
   TATGAATAAA TGACATTTTG TGCCCTGACC TTTGTTAGTT TAGGTTTAGA TTATCCTCTG  1560
   TTATTGACTT ATTGTGCTTT CTGGTTGTTA GGGTTTCTAA AAGACATAGT TGTTTATATA  1620
   TATGTCTGAC TTTGTATTCC GGATTTGGTT CTCTTGTGTC ATTAACTTGG GTTTAGCCAT  1680
   TATTACTTAA GAGTGGCAAC GAAATCAAAA AAAAAAAAAA AAAAAAAAAA AAAAAAA     1737
<210> 8
<211> 431
<212> amino acid
<213> Arabidopsis sp.
<220>
<223>
<223> topology, linear
<223> molecular type, deduced amino acid sequence of SEQ ID NO : 7
<400> 8
   Met Gly Thr His Ile Asn Phe Asn Asn Leu Gly Gly Gly Gly His Pro Gly
    1               5                  10                  15
   Gly Glu Gly Ser Ser Asn Gln Met Lys Pro Thr Gly Ser Val Met Pro Leu
           20                  25                  30
   Ala Arg Gln Ser Ser Val Tyr Ser Leu Thr Phe Asp Glu Leu Gln Asn Thr
   35                  40                  45                  50
   Leu Gly Gly Pro Gly Lys Asp Phe Gly Ser Met Asn Met Asp Glu Leu Leu
               55                  60                  65
   Lys Ser Ile Trp Thr Ala Glu Glu Ala Gln Ala Met Ala Met Thr Ser Ala
       70                  75                  80                  85
   Pro Ala Ala Thr Ala Val Ala Gln Pro Gly Ala Gly Ile Pro Pro Pro Gly
                   90                  95                  100
   Gly Asn Leu Gln Arg Gln Gly Ser Leu Thr Leu Pro Arg Thr Ile Ser Gln
           105                 110                 115
   Lys Thr Val Asp Glu Val Trp Lys Cys Leu Ile Thr Lys Asp Gly Asn Met
   120                 125                 130                 135
   Glu Gly Ser Ser Gly Gly Gly Gly Glu Ser Asn Val Pro Pro Gly Arg Gln
               140                 145                 150
   Gln Thr Leu Gly Glu Met Thr Leu Glu Glu Phe Leu Phe Arg Ala Gly Val
       155                 160                 165                 170
   Val Arg Glu Asp Asn Cys Val Gln Gln Met Gly Gln Val Asn Gly Asn Asn
                   175                 180                 185
   Asn Asn Gly Phe Tyr Gly Asn Ser Thr Ala Ala Gly Gly Leu Gly Phe Gly
           190                 195                 200
   Phe Gly Gln Pro Asn Gln Asn Ser Ile Thr Phe Asn Gly Thr Asn Asp Ser
   205                 210                 215                 220
   Met Ile Leu Asn Gln Pro Pro Gly Leu Gly Leu Lys Met Gly Gly Thr Met
               225                 230                 235
   Gln Gln Gln Gln Gln Gln Gln Gln Leu Leu Gln Gln Gln Gln Gln Gln Met
       240                 245                 250                 255
   Gln Gln Leu Asn Gln Pro His Pro Gln Gln Arg Leu Pro Gln Thr Ile Phe
                   260                 265                 270
   Pro Lys Gln Ala Asn Val Ala Phe Ser Ala Pro Val Asn Ile Thr Asn Lys
           275                 280                 285
   Gly Phe Ala Gly Ala Ala Asn Asn Ser Ile Asn Asn Asn Asn Gly Leu Ala
   290                 295                 300                 305
   Ser Tyr Gly Gly Thr Gly Val Thr Val Ala Ala Thr Ser Pro Gly Thr Ser
               310                 315                 320
   Ser Ala Glu Asn Asn Ser Leu Ser Pro Val Pro Tyr Val Leu Asn Arg Gly
       325                 330                 335                 340
   Arg Arg Ser Asn Thr Gly Leu Glu Lys Val Ile Glu Arg Arg Gln Arg Arg
                   345                 350                 355
   Met Ile Lys Asn Arg Glu Ser Ala Ala Arg Ser Arg Ala Arg Lys Gln Ala
           360                 365                 370
   Tyr Thr Leu Glu Leu Glu Ala Glu Ile Glu Lys Leu Lys Lys Thr Asn Gln
   375                 380                 385                 390
   Glu Leu Gln Lys Lys Gln Ala Glu Met Val Glu Met Gln Lys Asn Glu Leu
               395                 400                 405
   Lys Glu Thr Ser Lys Arg Pro Trp Gly Ser Lys Arg Gln Cys Leu Arg Arg
       410                 415                 420                     425
   Thr Leu Thr Gly Pro Trp
                   430

## Description

### BACKGROUND OF THE INVENTION

This specification relates to a family of novel transcription factors that bind to various abscisic acid responsive elements(ABREs), more particularily, factors named as ABFs(ABRE-Binding Factors) isolated by yeast one-hybrid screening of an*Arabidopsis* cDNA expression library using a prototypical ABRE (GGACACGTGGCG).
ABF3 is the subject of the present invention.

Abscisic acid (ABA) is one of the major plant hormones that plays an important role during plant growth and development (Leung and Giraudat, 1998). The hormone controls several physiological processes during seed development and germination. During vegetative growth, ABA is known to mediate responses to various adverse environmental conditions such as drought, high salt and cold/freezing (Shinozaki and Yamaguchi-Shinozaki, 1996).

One of the ABA-mediated responses to various environmental stresses is the induced expression of a large number of genes, whose gene products are involved in the plant's adaptation to the stresses (Ingram and Bartels, 1996). ABA responsive elements (ABREs), i.e., *cis*-regulatory elements that mediate the ABA-modulated gene expression, have been identified from the promoter analysis of ABA-regulated genes (reviewed in Busk and Pages, 1998). One class of the ABREs includes elements that share a PyACGTGGC (Py indicates C or T) consensus sequence, which can be considered a subset of a larger group of *cis*-elements known as "G-box" (Menkens *et al*., 1995). Another class of ABREs, known as "coupling elements (CE)" or "motif III", shares a CGCGTG consensus sequence. Both classes of ABREs, here, referred to as G/ABRE (G-box-like ABRE) and C/ABRE (CE-like ABRE), respectively, are almost ubiquitous in the promoter regions of ABA responsive genes of both monocotyledonous and dicotyledonous plants.

A number of basic leucine zipper (bZIP) class DNA-binding proteins are known to interact with the ABREs (Busk and Pages, 1998). EmBP1 and TAF1 have been isolated based on their *in vitro* binding activity to G/ABREs. GBF3, originally identified as one of the G-box binding factors (GBFs) involved in the light regulation of a ribulose bisphosphate carboxylase gene (Schindler *et al*., 1992), has been cloned using the ABA-responsive, G-box element of a *Arabidopsis Adh1* gene. Recently, a family of embryo-specific factors has been reported that can recognize both G/ and C/ABREs (Kim and Thomas, 1998; Kim *et al*., 1997). Other factors binding to G-box have also been described (Foster *et al*., 1994).

Although ABRE-binding factors have been known for some time, several observations suggest that hitherto unidentified factors are involved in ABA-regulated gene expression during stress response, especially in vegetative tissues. ABA-induction of rice *rab16A* and *Arabidopsis rd29B* genes requires *de novo* protein synthesis (Nakagawa *et al*., 1996; Yamaguchi-Shinozaski and Shinozaki, 1994), suggesting the involvement of ABA-inducible factors. *In vivo* binding of ABA-inducible factors has been demonstrated in maize *rab17* gene (Busk *et al*., 1997). In the case of *rab16B* gene, currently unknown, C/ABRE-binding factor(s) has been suggested to mediate ABA response through the motif III (Ono *et al*., 1996). Furthermore, it has been well established by genetic studies that different ABA signaling pathways operate in seeds and in vegetative tissues, respectively (Leung and Giraudat, 1998), and tissue-specific ABRE-binding activities have been demonstrated (Pla *et al*., 1993). None of the source materials used in the previous protein-DNA interaction clonings, however, were ABA- or stress-treated young plant tissues, and thus, inducible factors that may be critical for the ABA-mediated stress response during vegetative growth phase may have been missed so far.

Numerous stress responsive genes involved in plant's adaptation to various environmental stresses are regulated by ABA through G/ABREs or C/ABREs (Ingram and Bartels, 1996). Therefore, overexpression of ABRE-binding transcription factors will result in the activation of these stress-inducible genes and thus enhanced stress tolerance. Hence, once isolated, the ABRE-binding factors will be suitable for the generation of transgenic plants that are tolerant to multiple environmental stresses. Feasibility of manipulating transcription factors for the improved stress tolerance has been demonstrated by others recently (Jaglo-Ottosen *et al*., 1998; Kasuga *et al*., 1999).

### SUMMARY OF THE INVENTION

This specification relates to a family of novel transcription factors that bind to various ABREs. The factors, named as ABFs (ABRE-Binding Factors), were isolated by yeast one-hybrid screening of an *Arabidopsis* cDNA expression library using a prototypical ABRE (GGACACGTGGCG). ABFs are bZIP class transcription factors that can bind to both G/ABREs and C/ABREs. Expression of ABFs is inducible by ABA and various stress treatments, and they can transactivate an ABRE-containing reporter gene in yeast. Thus, ABFs have potential to activate a large number of ABA/stress responsive genes and thus can be used to generate transgenic plants that are tolerant to multiple environmental stresses.

### BRIEF DESCRIPTION OF THE DRAWINGS

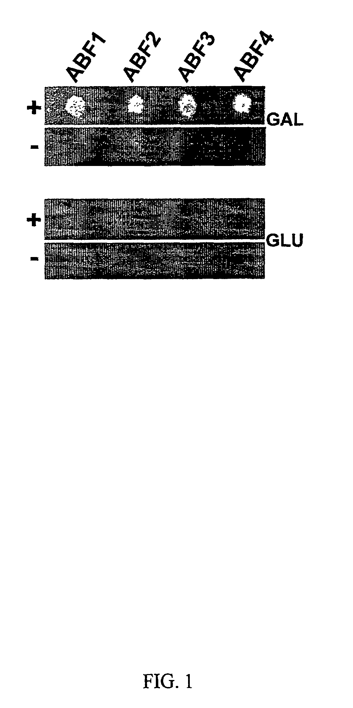

FIG. 1. **Specificity of ABF binding.** Binding specificity of ABFs. A reporter yeast containing a HIS3 reporter construct with (+) or without (-) the ABRE (a trimer of Em1a) was transformed with DNA from representative clones, and transformants were grown on either galactose (GAL) or glucose (GLU) medium lacking histidine.

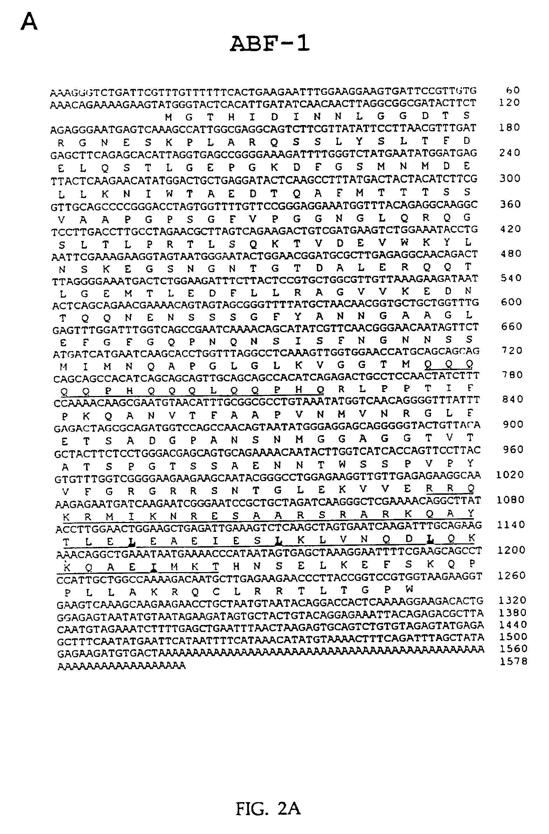

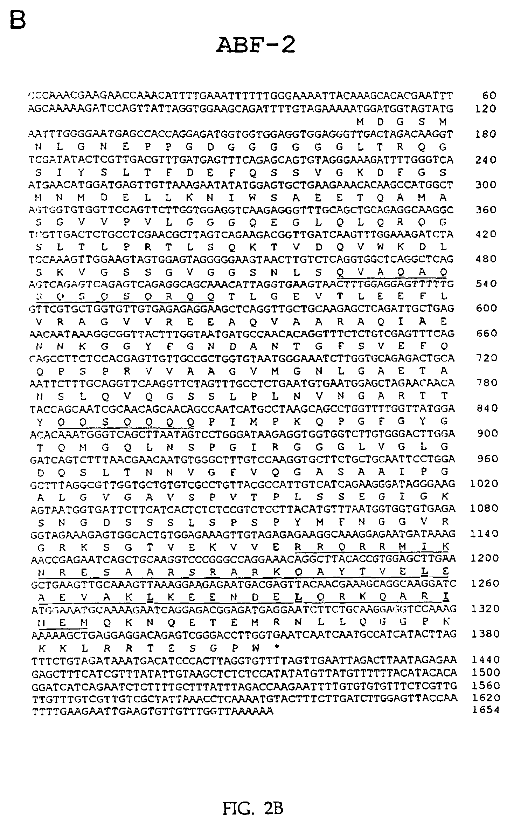

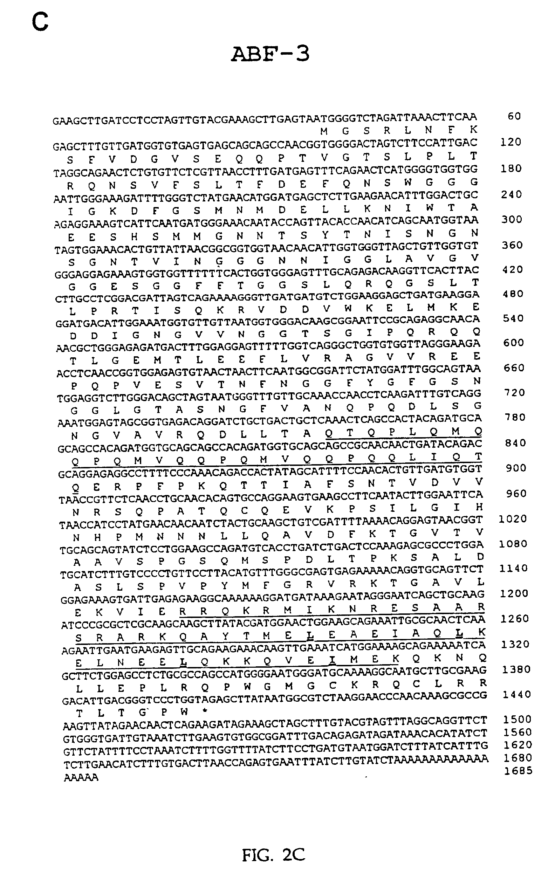

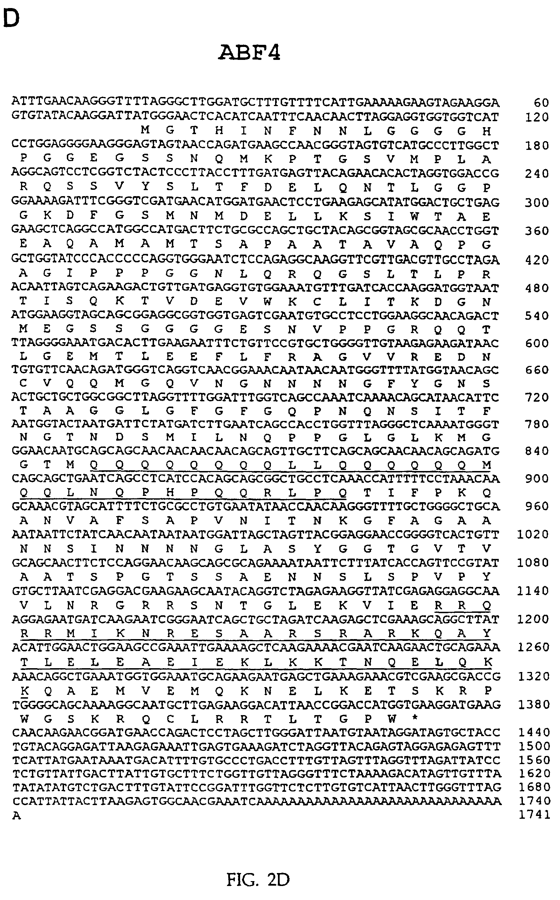

FIG. 2. **Nucleic and deduced amino acid sequences of ABFs**. Nucleic acid sequences of ABFs are presented together with deduced amino acid sequences. The bZIP and the glutamine-rich regions are underlined. ABF1, 2, 3 and 4 correspond to clones 1, 2, 11 and 19, respectively.

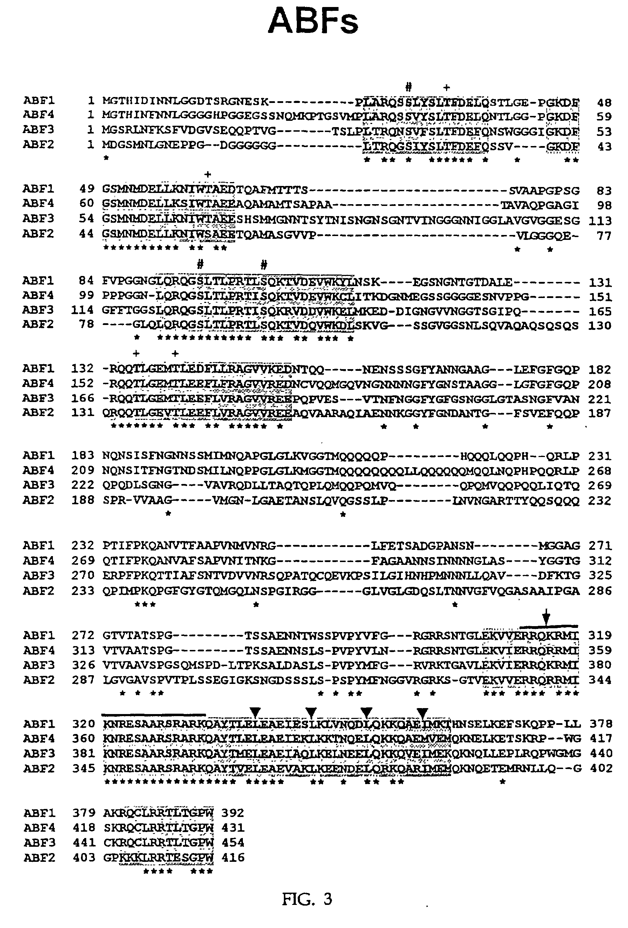

FIG. 3. **Alignment of the deduced amino acid sequences of ABFs.** The deduced amino acid sequences of ABFs are aligned together. The basic region and the leucine repeats are shown by a thick line and arrowheads, respectively. The small arrow indicates the arginine and the lysine residues within the basic regions that are discussed in the text. Regions highly conserved are highlighted. #, CaMK II sites. +, CK II sites.

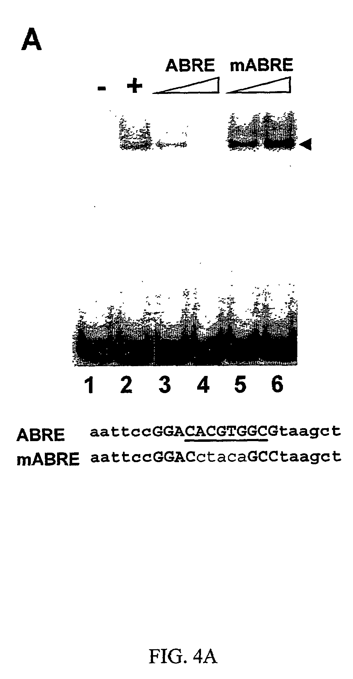

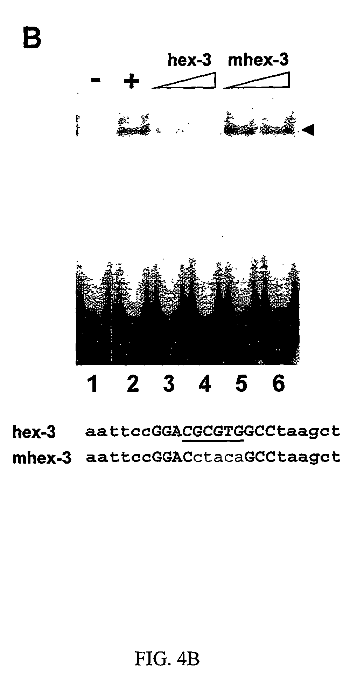

FIG. 4. **Electrophoretic mobility shift assay.** *A*, ABF1 binding to a G/ABRE. An oligonucleotie (ABRE) containing the Em1a element was employed as a probe in a mobility shift assay. *B*, ABF1 binding to a C/ABRE. An oligonucleotide containing the *hex-3* sequence was employed as a probe. In each assay, 1 µg of recombinant ABF1 was used. Lanes 1, probe only (-); 2, probe and ABF1 (+); 3 and 4, 100-fold and 200-fold molar excess of specific competitors (ABRE and *hex-3*), respectively; 5 and 6, 100-fold and 200-fold molar excess of a mutated oligonucleotide (mABRE and *mhex-3*) as competitors, respectively. Sequences of oligonucleotides are shown at the bottom of each figure and shifted bands are indicated by arrowheads.

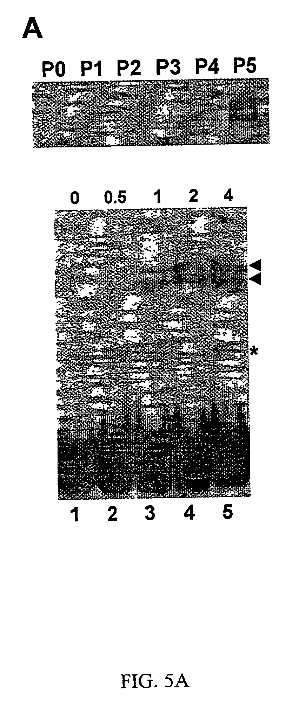

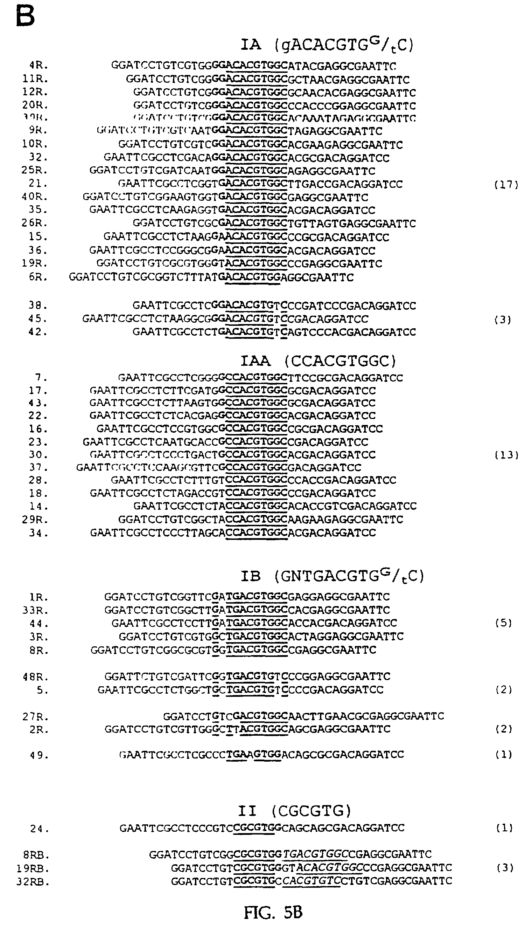

FIG. 5. **Binding site selection assay**. *A*, binding site selection assay. *Top*; Probes (P0 to P5) after each round of selection were amplified and used in EMSA. 1.5 µg of ABF1 was used. Only the top part of the gel containing the shifted bands is shown. *Bottom*; EMSA of P5 probe DNA. P5 probe DNA was employed in EMSA and titrated with increasing amount (µg) of ABF1. Arrowheads denote shifted bands. The band shown by * is probably an artifact resulting from secondary structure formation of palindromic sequences in the selected sequences. *B*, selected sequences. The selected sequences are aligned and grouped according to their consensus sequences shown in parentheses. The nucleotides highly conserved within each group are in bold, and those 100% conserved are underlined. G/ABRE elements flanking the C/ABRE core of group II sequences are in italics and underlined. The number of selected sequences in each group is indicated in the parentheses on the right.

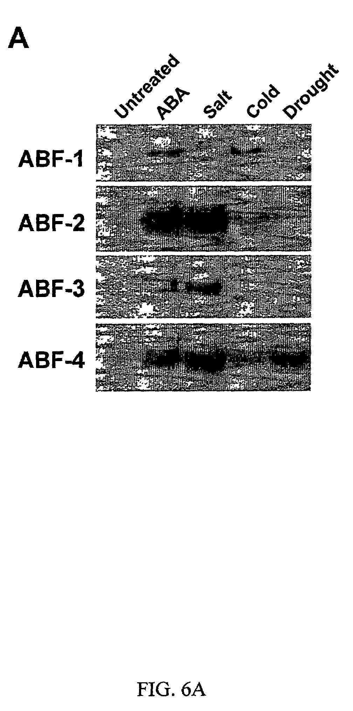

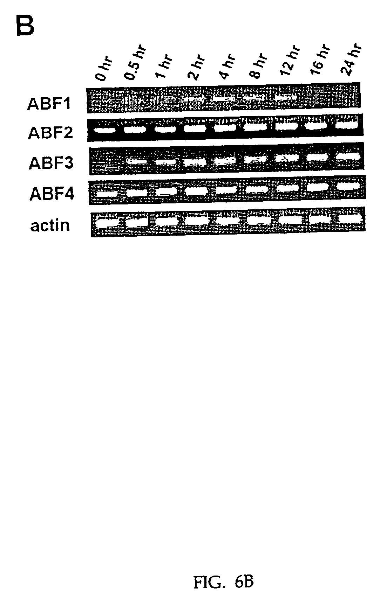

FIG. 6. **Analysis of ABF expression.** ABA- and stress-inducibility of ABF expression were examined by RNA gel blot analysis or RT-PCR. *A*, inducibility of ABF expression. 25 µg of total RNAs isolated from untreated plants or plants treated with ABA, high salt, cold or drought, were transferred to a membrane and probed with specific probes. *B*, time course of ABA-induction. RT-PCR reactions were performed using 0.5 µg total RNAs from plants treated with 100 µM ABA for 0 min, 30 min, 1 hr, 2 hr, 4 hr, 8 hr 12 hr, 16 hr, and 24 hr. actin, a control reaction performed with an actin gene of *Arabidopsis thaliana*.

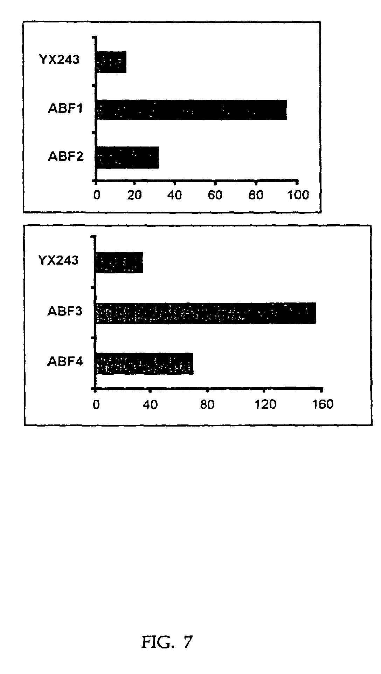

FIG. 7. **Transactivation assay of ABFs.** Transactivational function of ABFs was tested by using a yeast system. ABFs were expressed in yeast that harbored an ABRE-containing *lacZ* reporter gene. The β-galactosidase activity was then assayed and indicated as Miller units. For each construct, 5 different transformants were assayed in duplicates. YX243, control vector without any inserts.

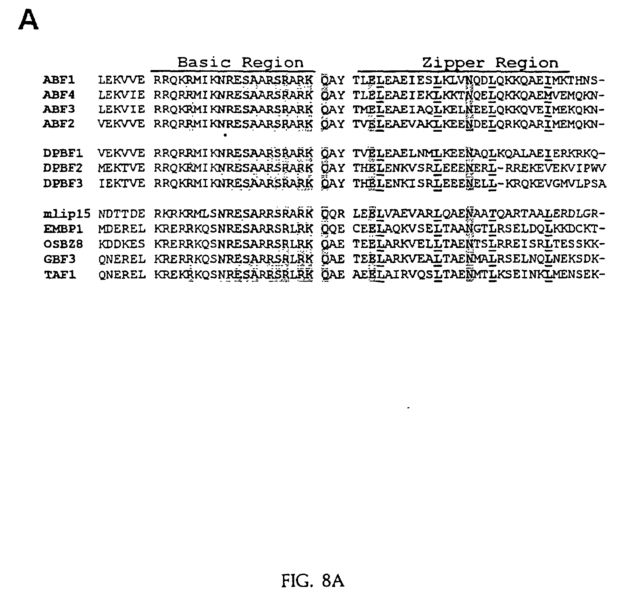

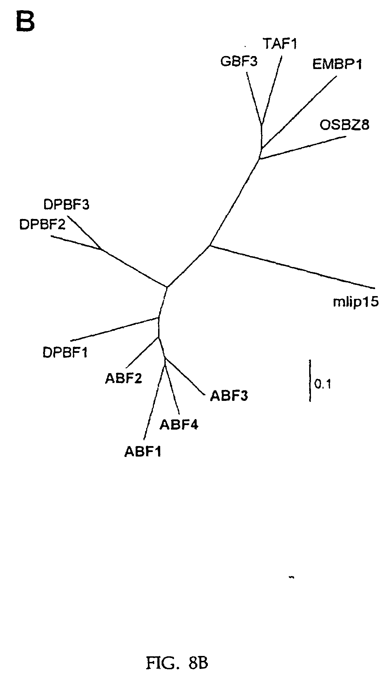

FIG. 8. **Phylogenetic analysis of ABRE-binding bZIP factors.** *A*, bZIP regions of the ABRE-binding factors mentioned in the text are aligned together. mlip15 is a maize bZIP factor induced by low-temperature (Kusano *et al*., 1995). Conserved amino acids are highlighted, and the leucine residues in the "zipper" regions are undelined. *B*, unrooted phylogenetic tree diagram. The bZIP regions shown in *A* were aligned and a tree diagram was constructed using CLUSTAL W algorithm.

### DETAILED DESCRIPTION OF THE INVENTION

### Materials and methods

*Plant materials―Arabidopsis thaliana* (ecotype Columbia) was grown at 22 °C on pots of soil (a 1:1 mixture of vermiculite and peat moss) irrigated with mineral nutrient solution (0.1% Hyponex) in 8 hr light/16 hr dark cycles. For RNA isolation, 4-5 weeks old plants were subject to various treatments, flash-frozen in liquid nitrogen and kept at -70 °C until needed. For ABA treatment, roots of plants were submerged, after the removal of soil, in a 100 µM ABA (Sigma, No A 1012) solution for 4 hr with gentle shaking. ABA solution was also sprayed intermittently during the incubation period. Salt treatment was performed in the same way, except that 250 mM NaCl solution was employed. For drought treatment, plants were withheld from water for two weeks before harvest, and left on the bench, after removing soil, for 1 hr just before collection. For cold treatment, plants were placed at 4 °C for 24 hr under dim light before harvest.

*Yeast techniques, DNA manipulation and RNA gel blot analysis*―Standard methods (Ausubel *et al*., 1994; Guthrie and Fink, 1991; Sambrook *et al*., 1989) were used in manipulating DNA and yeast. DNA sequencing was performed on ABI 310 Genetic Analyzer, according to the manufacturer's instruction. DNA sequence analysis was done with DNA Strider® and Generunr®, and BLAST algorithm (Altschul *et al*., 1990) was used for database search. Multiple sequence alignment and phylogenetic tree construction were performed with CLUSTAL W program (Thompson *et al*., 1994) available on the web (http://www2.ebi.ac.uk/clustalw).

RNA was isolated according to Chomczynski and Mackey (1995) and further purified by LiCl precipitation followed by ethanol precipitation. For RNA gel blot analysis, 25 µg of total RNA was fractionated on 1.1% formaldehyde agarose gel, transferred to nylon membrane (Hybond-N+, Amersham) by "downward capillary transfer" method (25), and fixed using Stratagene's UV Crosslinker (Model 2400). Loading of equal amount of RNAs was confirmed by ethidium bromide staining. Hybridization was at 42 °C in 5X SSC (1X SSC is 0.15 M NaCl, 0.015 M sodium citrate), 5X Denhardt's solution (1X Denhardt's solution is 0.02% Ficoll, 0.02% PVP, 0.02% BSA), 1% SDS, 100 µg/ml salmon sperm DNA, and 50% foramide for 24-30 hrs. Probes were prepared from the variable regions of ABFs. After hybridization, filters were washed twice in 2X SSC, 0.1% SDS at room temperature and three times in 0.2X SSC, 0.1% SDS for 10 min each at 65 °C. Exposure time was 7-8 days. RT-PCR was performed employing the Access RT-PCR System (Promega) using 0.5 g of total RNA according to the manufacturer's instruction. Amplification after the first strand cDNA synthesis was 45, 35, 40 and 45 cycles for ABF1, 2, 3 and 4, respectively. ABF primers (sequences are available upon request) were from variable regions between the bZIP and the conserved regions. The actin primers used in the control reaction was from the *Arabidopsis* actin-1 gene (GenBank Accession No., M20016). Free of contaminating DNA in RNA samples was confirmed by using primer sets (ABF3 and actin) that flank introns.

*cDNA library construction and yeast one-hybrid screening*―Poly A(+) RNA was isolated from total RNAs prepared from ABA- or salt-treated *Arabidopsis* seedlings, using Qiagen's Oligotex resin. cDNA was synthesized from an equal mixture (6 µg total) of poly A(+) RNAs prepared from the two sources of total RNAs employing a Stratagene's cDNA synthesis kit. cDNA was fractionated on a Sepharose CL-2B column, peak fractions containing cDNAs larger than 500 bp were pooled, and pooled cDNAs were ligated with pYESTrp2 (Invitrogen) predigested with *Eco* RI-*Xho* I. The ligation mixture was electroporated into *E.coli* DH10B cells. Titer of this original library was 5.4 x 10⁷ cfu. Portion of the library (2 x 10⁷) was plated on 15 cm plates at a density of 150,000 cfu/plate. Cells were suspended in LB after overnight growth at 37 °C on plates and pooled together. Finally, plasmid DNA was prepared from the collected cells.

pYC7-I and pSK1 (Kim and Thomas, 1998) were used as *HIS3* and *lacZ* reporter plasmids, respectively. The G/ABRE reporter construct was prepared by inserting a trimer of Em1a element (GGACACGTGGCG; Guiltinan *et al*., 1990) into the *Sma* I site of pYC7-I and the *Xba* I site of pSK1. In order to prepare reporter yeast, YPH 500 was transformed with the *Stu* I-digested pYC7-I reporter construct. Resulting Ura+ colonies were transformed with the pSK1 construct and maintained on a SC-LEU-URA medium. Screening of the library was performed as described (Kim and Thomas, 1998) except that transformed reporter yeast was grown on Gal/Raf/CM-HIS-LEU-TRP plates instead of Glu/CM-HIS-LEU-TRP plates. Putative positive clones from the screen were streaked on fresh Gal/Raf/CM-HIS-LEU-TRP plates to purify colonies. After β-galactosidase assay, well-isolated single colonies were patched on Glu/CM-LEU-TRP-URA plates to be kept as master plates. Galactose- dependency of the His⁺/*lac*Z⁺ phenotype of the purified isolates was examined subsequently by comparing their growth pattern and β-galactosidase activity on Gal/Raf/CM-HIS-LEU-TRP and Glu/CM-HIS-LEU-TRP dropout plates.

*Analysis of positive clones―*Yeast DNA was prepared from 1.5 ml of overnight cultures of the positive clones. PCR was performed with primers derived from the pYESTrp2 vector sequences flanking the inserts (pYESTrp forward and reverse primers). PCR products were digested with *Eco* RI, *Hae* III, or *Alu* I in order to group the cDNAs. For library plasmid rescue, yeast DNAs from representative clones were introduced into DH10B *E.coli* cells by electroporation. Plasmid DNAs used in DNA sequencing and confirmation experiments were isolated from these *E.coli* transformants by the alkaline lysis method. For the confirmation experiment shown in Fig. 1, plasmid DNAs thus isolated were re-introduced into the yeast containing pSK1 or ABRE-pSK1, transformants were kept on Glu/CM-LEU-TRP plates, and their growth was tested after spotting 5 µl of overnight cultures (1/50 dilutions) on Gal/Raff/CM-HIS-LEU-TRP or Glu/CM-HIS-LEU-TRP plates containing 2.5 mM 3-aminotriazole.

*Isolation of full-length ABF3 and 4*―A PCR approach was used to isolate the missing 5' portions of clone 11 and clone 19. Database search revealed that clone 11 was part of the BAC clone F28A23 of the *Arabidopsis* chromosome IV. On the other hand, the 5' portion of the clone 19 sequence was identical to the 3' region of an EST clone, 176F17T7. Based on the sequence information, 5' PCR primers (5'-GAAGCTTGATCCTCCTAGTTGTAC-3' for clone 11 and 5'-ATTTGAACAAGGGTTTTAGGGC-3' for clone 19) were synthesized. 3' primers (5'-TTACAATCACCCACAGAACCTGCC-3' and 5'-GATTTCGTTGCCACTCTTAAG-3', which are complementary to the 3'-most sequences of clones 11 and 19, respectively) were prepared using our sequence information. PCR was performed with *Pwo* polymerase (Boeringer Mannheim), using the primer sets and 1 µg of our library plasmid DNA. After 30 cycles of reaction, the DNA fragments corresponding to the expected size of the full-length clones were gel-purified and cloned into the PCR-Script vector (Stratagene). Several clones from each PCR product were then sequenced in their entirety. The fidelity of the full-length sequences was confirmed by comparing their sequences with each other and with those of the original partial clones and the genomic clones deposited later by the Arabidopsis Genome Initiative Project.

*Plasmid constructs―*In order to prepare a GST-ABF fusion constructs, entire coding regions and the 3' untranlated regions of ABF1 and ABF3 were amplified by PCR using *Pfu* polymerase (Stratagene). After *Xho* I digestion followed by gel-purification, the fragments were cloned into the *Sma* I-*Sal* I sites of pGEX-5X-2 (Pharmacia Biotech). The constructs used in transactivation assay were also prepared in a similar way. The coding regions were amplified by PCR. Resulting fragments were digested with *Xho* I, gel-purified and cloned into pYX243. pYX243 was prepared by *Nco* I digestion, Klenow fill-in reaction, *Sal* I digestion and gel-purification. Intactness of the junction sequences was confirmed by DNA sequencing.

*Preparation of recombinant ABFs and mobility shift assay*―Recombinant ABF1 and ABF3 were prepared employing a GST Purification Module from Pharmacia Biotech, according to the supplier's instruction. *E.coli* BL21 cells were transformed with the GST-ABF constructs by electroporation. In order to prepare bacterial extract, a single colony of transformed bacteria was inoculated in 2YT/Amp medium and grown overnight. The culture was diluted (1:100) into 250 ml of fresh media. IPTG was added to the culture to a final concentration of 0.1 mM when A₆₀₀ reached 0.7. Cells were harvested by centrifugation after further growth (1.5 hr). The bacterial pellet was resuspended in 12.5 ml of PBS (0.14 M NaCl, 2.7 mM KCl, 10.1 mM Na₂HPO₄, 1.8 mM KH₂PO₄, PH7.3) and sonicated on a Branson Sonifier 250 (4 x 40 s burst at setting 5 at 80% duty cycle). The lysate was cleared of cell debris by centrifugation, and the supernatant was loaded onto a column packed with 0.125 ml (bed volume) of glutathione Sepharose 4B resin. Wash and elution was performed as suggested by the supplier. Protein concentration was determined using the BIO-RAD protein assay kit. Production of GST-ABF1 fusion protein was confirmed by Western blotting using GST antibody.

Mobility shift assay was performed as described (Kim *et al*., 1997). To prepare probes, oligonucleotide sets shown in Fig. 4 were annealed by boiling 100 pmoles each of complementary oligonucleotides for 5 min and slowly cooling to room temperature. Portions of the annealed oligonucletides (4 pmoles of each set) were labeled by Klenow fill-in reaction in the presence of ³²P-dATP. Binding reactions were on ice for 30 min, and electrophoresis was performed at 4 °C.

*Binding site selection assay*―A pool of 58 bases oligonucleotide, R58, containing 18 bases of random sequence was synthesized: CAGTTGAGCGGATCCTGTCG(N)₁₈GAGGCGAATTCAGTGCAACT. The random sequence is flanked by *Bam* HI and *Eco* RI sites for the convenience of cloning after selection. R58 was made double strand by annealing a primer, RANR, AGTTGCACTGAATTCGCCTC, and then by extending it using Klenow fragment. For the first round of selection, 5 pmoles of the double strand R58 (P0 probe) was mixed with 5 µg of the recombinant ABF1 in a 100 µl of binding buffer (10% glycerol, 25 mM HEPES, pH 7.6, 100 mM NaCl, 0.5 mM EDTA, 1 mM DTT) containing 4 µg of poly [d(I-C)] and incubated on ice for 30 min. The mixture was loaded onto 0.1 ml of glutathione Sepharose 4B resin packed on a disposable column, washed with 10 volumes of the binding buffer, and eluted with 0.3 ml of 10 mM glutathione. Bound DNA was purified by phenol/chloroform extraction followed by ethanol precipitation. Amplification of the selected DNA was performed by PCR, using 20 pmoles each of RANF (CAGTTGAGCGGATCCTGTCG) and RANR primers in a buffer (10 mM Tris, pH 9.0, 50 mM KCl, 0.1% Triton X-100, 2.5 mM MgCl₂) containing 150 µM dNTP-dATP, 4 µM dATP, 10 µCi of ³²P-dATP. Reaction was carried out 20 cycles (10 sec, 94 °C/10 sec, 50 °C/1 min, 72 °C). Amplified DNA was purified on a polyacrylamide gel, the band was excised after autoradiography, and DNA was eluted by standard method to be used as a probe DNA for the next round of selection. The selection cycle was repeated two more times. For the fourth and the fifth rounds of selection, bound DNAs were isolated after EMSA, by eluting DNA from the dried gel fragment containing the shifted bands. The amplified DNA (P5 probe) from the last selection was cloned into pBluescript (Stratagene) after *Eco* RI and *Bam* HI digestion, and plasmid DNAs from 50 random colonies were sequenced.

*Transactivation assay*―Reporter yeast containing the *lacZ* reporter gene (pYC7-I) with or without the ABRE was transformed with various pYX243/ABF constructs, and transformants were kept on Glu/CM-LEU-URA plates. For the assay, 5 colonies from each transformant group were grown in a Glu/CM-LEU-URA medium overnight to A₆₀₀ of approximately 1. The cultures were diluted 4-6 times with fresh media, grown further for 3 hr, and pelleted by brief centrifugation. The cells were washed twice with Gal/Raf/CM-LEU-URA medium, resuspended in 4 ml of the same medium, and grown for 4 hr to induce the expression of ABFs. A₆₀₀ was measured at the end of the growth period, and 0.5 ml aliquots of the culture, in duplicates, were pelleted. The pellets were resuspended in 0.665 ml of H buffer (100 mM HEPES, 150 mM NaCl, 2mM MgCl₂, 1% BSA, pH 7.0) and permeabilized by vortexing for 1 min after the addition of 0.055 ml each of CHCl₃ and 0.1% SDS. The reaction was started by adding 0.125 ml of 40 mM stock solution of CPRG (chlorophenylred-b-D-galactopyranoside) and incubation was continued at 30 °C until the color changed to red. Reactions were stopped by the addition of 0.4 ml of 1 M Na₂CO₃. The mixtures were microfuged for 5 min to remove cell debris and A₅₇₄ was measured. β-galactosidase activity was expressed in Miller units.

### Results

*Isolation of ABRE-binding protein factors―*We employed a modified yeast one-hybrid system (Kim and Thomas, 1998; Kim *et al*., 1997) in order to isolate ABRE-binding factor(s) using the prototypical ABRE, Em1a element (GGACACGTGGCG). A cDNA expression library representing 2 x 10⁷ cfu was constructed in a yeast expression vector pYESTrp2, using a mixture of equal amounts of mRNAs isolated from ABA- and salt-treated *Arabidopsis* plants. The vector contains B42 activation domain (Ma and Ptashne, 1987) whose expression is under the control of yeast GAL1 promoter. Thus, expression of cDNAs, which are inserted as a fusion to the activation domain, is inducible by galactose and repressed by glucose. The library DNA was used to transform a reporter yeast that harbors the ABRE-containing HIS3 and *lac*Z reporters. From a screen of 4 million yeast transformants, ca. 40 His⁺ blue colonies were obtained, among which 19 isolates were characterized further. Analysis of the cDNA inserts of the positive clones indicated that they could be divided into 4 different groups according to their restriction patterns. Representative clones with longer inserts from each group were analyzed in more detail.

First, binding of the cDNA clones to the G/ABRE in yeast was confirmed. The G/ABRE-HIS3 reporter yeast was re-transformed with the library plasmid DNAs isolated from the representative clones. Growth pattern of the transformants on media lacking histidine was then examined to measure the HIS3 reporter activity. The result in FIG. 1 showed that transformants obtained with all four clones could grow on a galactose medium lacking histidine, but not on a glucose medium. In the same assay, the transformed yeast containing a control reporter construct lacking the ABRE could not grow on the same galactose medium. Thus, the clones could activate HIS3 reporter gene reproducibly, indicating that they bind to the ABRE in yeast.

Next, nucleotide and deduced amino acid sequences of the representative clones were determined. Clone 1, which represents two isolates, contained a cDNA insert of 1578 bp including a poly A(+) tail (FIG. 2A, SEQ ID NO. 1). An open reading frame (ORF) that is in frame with the B42 domain was present within the sequence. The ORF, referred to as ABF1 (ABRE-Binding Factor 1), contains an ATG initiation codon near the B42-cDNA junction, suggesting that it is a full-length clone. The amino acid sequence starting from the initiation codon is shown in FIG. 2A (SEQ ID NO. 2). The insert of clone 2, which represents 8 isolates, is 1654 bp long (FIG. 2B, SEQ ID NO. 3) and the longest ORF including an initiation codon near the B42-cDNA junction encodes a protein of 416 amino acids (ABF2) (FIG. 2B, SEQ ID NO. 4).

The insert of clone 11, representing 6 isolates, encoded a protein containing 434 amino acids. An ORF containing 366 amino acids was found in clone 19 cDNA. The clones were partial, however, and the missing 5' portions were isolated using the available partial sequence information on databases (Materials and methods). Sequencing of the full-length clones (FIGs. 2C and 2D, SEQ ID NOs. 5 and 7) showed that the original clone 11 was missing the first 20 amino acids, and thus, full-length clone 11 encodes a protein containing 454 amino acids (ABF3) (FIG. 2C, SEQ ID NO. 6). The longest ORF of clone 19 is composed of 431 amino acids (ABF4) (FIG. 2D, SEQ ID NO. 8).

*ABFs are bZIP proteins*―Analysis of the deduced amino acid sequence of ABF1 revealed that it has a basic region near its C-terminus (FIGs. 2A and 3). The region immediately downstream of it contains 4 heptad repeats of leucine, indicating that ABF1 is a bZIP protein (Landshulz *et al.*, 1988). Similarly, other ABFs also have a basic region followed by a leucine repeat region (FIGs. 2B-2D and 3). The basic regions of ABF1 and ABF3 are identical to each other, and those of ABF 2 and ABF4 are also identical (FIG. 3). The two, shared basic regions are same except that one of the lysine residues of ABF1 and ABF3 is replaced by arginine in ABF 2 and ABF4. The analysis shows that a family of bZIP proteins with conserved basic regions interacts with the G/ABRE.

ABFs also share several highly conserved regions outside the basic domains. As shown in FIG. 3, the conserved regions are clustered in the N-terminal halves. Invariably, they contain one or two potential phosphorylation sites. The N-most region, for example, contains one multifunctional calmodulin-dependent protein kinase II (CaMK II) site (X-**R**-X-X-**S***-X) (Kemp and Pearson, 1990) followed by a caseine kinase II (CKII) phosphorylation site (X-**S**/**T***-X-X-**D**/**E**-X). One or two CaMK II or CK II phosphorylation sites are also present in other conserved regions. The middle portions of ABFs are highly variable and rich in glutamine commonly found in transcriptional activation domains.

*In vitro binding activity of ABFs*―In order to test *in vitro* DNA-binding activity of ABFs, we performed electrophoretic mobility shift assay (EMSA) using recombinant ABF1 or ABF3 and probe DNAs containing a G/ or C/ABRE. Similar results were obtained with both proteins and the assay result of ABF1 is shown in FIG. 4. A major shifted band was observed, with a weaker minor band (FIG. 4A, lane 2). Addition of excess, increasing amount of unlabeled probe DNA to the reaction mixture (lanes 3 and 4) gradually abolished the binding, whereas the same amount of a mutated oligonucleotide (lanes 5 and 6) did not. Thus, ABF1 and ABF3 exhibited sequence-specific binding activity to the G/ABRE *in vitro*.

ABFs are similar to the *Dc3* promoter-binding factors (DPBFs) (Kim and Thomas, 1998; Kim *et al*., 1997) in their basic regions (see Discussion). Since DPBFs are known to interact not only with a G/ABRE but also with C/ABREs, we tested whether ABFs can interact with C/ABREs. To date, no transcriptional activators interacting with the element were reported except the embryo-specific DPBFs. An oligonucleotide, *hex-3* (Lam and Chua, 1991), containing the C/ABRE core sequence (CGCGTG), was employed as a probe in an EMSA. As shown in FIG. 4B, a shifted band was observed (lane 2). The band formation was abolished by the addition of excess amounts of the cold probe DNA to the reaction mixture (lanes 3 and 4). The competition was not observed with a mutated probe DNA (lanes 5and 6), demonstrating that the binding was specific to the C/ABRE. Thus, ABF1 and ABF3 could bind to a C/ABRE as well.

*Binding site preference of ABF1―*Our *in vitro* binding assay indicated that ABF1 and ABF3 can interact with both G/ and C/ABREs, although mutual competition assay (not shown) showed that they have higher affinity to the G/ABRE. In order to investigate ABF binding sites further, we performed a random binding site selection assay (RBSA) (Pollock and Treisman, 1990) (Materials and methods), using the recombinant ABF1. Shifted bands were visible on a mobility shift assay gel after three rounds of selection (FIG. 5A, top panel). After confirming the binding of ABF1 to the probe DNAs from the final round of selection (FIG. 5A. bottom panel), the DNAs were cloned and sequenced.

The 44 selected sequences are presented in FIG. 5B. The sequences could be divided into 4 groups (groups IA, IAA, IB, and II) according to their consensus sequences. All of the group I sequences, except one (sequence no. 49), contain an ACGT element, while the group II sequences contain the C/ABRE core. The most frequently selected sequences (30 of 44) are those sharing a strong G/ABRE, CACGTGGC (Busk and Pages, 1998): gACACGTGGC (group IA) or CCACGTGGC (group IAA). The group IAA element is similar to the prototypical ABRE, Em1a (GGACACGTGGC), while the group IAA consensus is the same as the palindromic G/ABREs present in many ABA-inducible genes such as maize *rab28*, *Arabidopsis kin1, cor6.6* and *Adh1* genes (reviewed in Thomas *et al*., 1997). In some of the group IA sequences (sequence no. 38, 45 and 42), the GGC following the ACGT core is replaced by GTC, forming another palindromic consensus sequence, GACACGTGTC. The group IB sequences share a GNTGACGTGGC consensus or its variants differing in one or two bases flanking the ACGT core. Although the conserved element differs from those of group IA and IAA in the bases preceding the ACGT core, it contains the same ACGTG(G/t)C. Hence, the preferred binding sites of ABF1 can be represented as ACGTG(G/t)C, with AC, CC or TG preceding it.

One of the selected sequences (no.24 of group II) contains the C/ABRE core sequence (CGCGTG). The three other group II sequences also contain the C/ABRE core. The element in them, however, is flanked by one of the group I consensus sequences, and thus, they contain both types of ABREs. Another sequence (no. 49 of group IB) does not contains the ACGT core; the C of the ACGT is replaced by A. The resulting AAGTGGA sequence is similar to the half G-box (CCAAGTGG) of *Arabidopsis Adh1* promoter, which is required for high level ABA-induction of the gene (de Bruxelles *et al*., 1996). Thus, ABF1 interacts with sequences without the ACGT core, which includes the C/ABRE. The low selection frequency, however, suggests that ABF1's affinity to them is lower.

*Expression of ABFs is ABA-inducible*―Since we are interested in ABA-inducible stress responsive factors, we investigated ABA-inducibility of ABF expression, by RNA gel blot analysis (FIG. 6A). With the ABF1 probe, no hybridization signal was detectable with RNA isolated from untreated plants, while a clear signal was detected with RNA from ABA-treated plants. Similar results were obtained with other ABF probes; while hybridization signals were weak (ABF2 and 4) or undetectable (ABF-3) with the RNA from untreated plants, distinct signals were observed with the RNA sample from ABA-treated plants. Thus, expression of ABFs is ABA-inducible.

Although all are induced by ABA, the time course of ABA-induced expression of ABFs was not identical to each other (FIG. 6B). ABF1 RNA level reached a peak approximately 2 hours after ABA treatment started, remained same up to 12 hours and decreased to the uninduced level after 16 hours. ABF2 and ABF4 expression appeared to be induced faster, reaching a plateau after 30 min of ABA treatment. Afterwards, their RNA level remained relatively same until 24 hour. The induction pattern of ABF3 was similar to those of ABF2 and ABF4 except that it reached the peak level later, i.e., after 2 hours.

We also examined the effect of various environmental stresses on the expression of ABFs. The results (FIG. 6A) showed that expression of ABF1 was induced by cold treatment, but not by other stress treatments. With the same RNA samples, ABF2 and ABF3, on the other hand, were not induced by cold, but by high salt treatment. ABF4 expression was induced by all three treatments, although induction level after cold treatment was relatively low. Expression of ABFs is, thus, inducible also by various environmental stresses and their induction patterns are differential, suggesting that they function in different stress responsive pathways.

*ABFs can transactivate an ABRE-containing reporter gene in yeast―*Our result so far demonstrated that ABF1, and probably other ABFs too, can bind to various ABREs and that their expression is both ABA- and stress-dependent. Thus, ABFs have potential to activate a large number of ABA/stress responsive genes, if they have transactivation capability. We therefore investigated whether ABFs can activate an ABRE-containing reporter gene. Coding regions of ABFs were cloned into a yeast expression vector and the constructs were individually introduced into a yeast strain that harbored a G/ABRE-containing *lacZ* reporter gene integrated into the chromosome. Subsequently, reporter enzyme activity was measured.

With the ABF1 construct, β-galactosidase activity was 6 times higher than that obtained with the control construct (FIG.7, top panel). No enzyme activity was detectable with the same ABF1 construct when a reporter lacking the ABRE was used. Thus, ABF1 can transactivate the reporter gene and the activation is ABRE-dependent. With the ABF2 construct, reporter enzyme activity two times higher than the background activity was detected, indicating that the factor also can transactivate the reporter gene (FIG. 7, top panel). Likewise, ABF3 and 4 could transactivate the reporter gene (FIG. 7, bottom panel). The activation level of ABF3 was higher than the ABF1's, while ABF4 showed weaker activation. The result of our transactivation assay demonstrates that ABFs can activate an ABRE-containing gene in yeast.

### Discussion

Numerous studies, both genetic and biochemical, show that ABA mediates stress response in vegetative tissues, although not all stress responses are ABA-dependent (Leung and Giraudat, 1998; Shinozaki and Yamaguchi-Shinozaki, 1996; Thomashow, 1998; Ingram and Bartels, 1996). Central to the response is the ABA-regulation of gene expression through G/ABREs or C/ABREs. Transcription factors mediating ABA-independent cold and drought responses have been reported recently (Jaglo-Ottosen *et al*., 1998; Liu *et al*., 1998). However, those regulating ABA-dependent stress response via the G/ or the C/ABREs have yet to be identified. Among the ABRE-binding factors mentioned earlier, TAF-1 is known not to be directly involved in ABA responsive gene expression (Oeda *et al*., 1991), while EmBP-1 and DPBFs are highly embryo-specific (Kim and Thomas, 1998; Hollung *et al*., 1997). GBF3 and a homology-based cloned factor OSBZ8, although inducible by ABA, are from cultured cells or from embryos (Lu *et al*., 1996; Nakagawa *et al*., 1996). Taken together with the lack of data demonstrating their role in ABA or stress response, it is likely that unknown factors may mediate ABA-responsive gene expression in vegetative tissues.

In a search for such transcription factors, we isolated a family of G/ABRE-binding proteins from young *Arabidopsis* plants treated with ABA or high salt. The factors, referred to as ABFs, are ABA/stress-inducible bZIP class transcription factors with shared basic regions. Sequence comparison with known ABRE-binding factors indicated that, although they do not show any significant homology to other factors, they are similar to the *Dc3* promoter-binding factors (DPBFs) (Kim and Thomas, 1998; Kim *et al*., 1997). DPBFs have been isolated from a seed-specific library based on their interaction with a *lea* gene promoter containing both G/ and C/ABREs. The two family members are nearly identical in their basic regions (FIG. 8A), and their DNA-binding properties are similar in that they can interact with both types of ABREs. Some of the conserved phosphorylation sites within the N-terminal halves of ABFs are also conserved in DPBFs. However, ABFs diverge from the DPBFs outside the basic regions and their immediate flanking sequences, overall identity being in the range of 30-40%. As a result, they form a subfamily distinct from DPBFs and also from other known factors, as shown in FIG. 8B. Furthermore, their expression patterns are different from those of DPBFs; i.e., DPBFs' expression is embryo-specific. Cloning of ABFs shows that two related subfamilies of ABRE-binding factors are present in seed and in vegetative tissues, respectively. The presence of distinct factors in the tissues that have similar ABRE-binding affinity has been demonstrated in maize (Pla *et al*., 1993).

ABFs contain regions highly conserved among them apart from the basic regions. Thus, ABFs appear to share some properties other than DNA-binding activity. The conserved regions, however, do not have any easily recognizable motifs except that two of them can form α-helix, and thus, their function remains to be identified. They may be involved in nuclear translocation, DNA-binding, transcriptional activation, or interaction with other regulatory proteins. Whatever their function may be, the conservation of potential phosphorylation sites within the regions suggests that it is probably modulated by post-translational modification.

Our *in vitro* binding assay showed that the most preferred binding site of ABF1 *in vitro* can be represented as CACGTGGC (FIG. 5B). The element, first identified as EmBP-1 recognition site (Guiltinan *et al*., 1990), are highly conserved among ABA/stress inducible promoters and strongly affect ABA-inducibility *in vivo* (Busk and Pages, 1998). Together with the fact that ABF1 is ABA/stress-inducible and has transcriptional activity, this suggests that ABF1 can potentially activate a large number of ABA/stress responsive genes. Also, ABF1 can bind to other ABREs including the C/ABREs, further supporting the broad spectrum of potential ABF1 target genes. The affinity to C/ABREs, however, was relatively low *in vitro*.

The expression pattern of ABFs suggests that each ABF is likely to be involved in different stress signaling pathways. Although all are ABA-inducible and can bind to same ABREs, they are differentially regulated by various environmental stresses. ABF1 expression is induced by cold, ABF 2 and ABF3 by high salt, and ABF4 by cold, high salt and drought. The simplest interpretation of the result would be that ABF1 is involved in cold signal transduction, while ABF2 and ABF3 function in osmotic stress signaling. ABF4, on the other hand, appears to participate in multiple stress responses. In addition, ABFs differ in their ABA induction patterns. Expression of ABF1 was induced rather slowly (FIG. 6B) and the accumulation of its RNA was transient, while induction of other ABFs appeared faster and their RNA levels remained relatively stable once reached a plateau. The multiplicity of ABA-dependent stress signaling pathways has been demonstrated in *Arabidopsis* by genetic analysis (Leung and Giraudat, 1998; Ishitani *et al*., 1997). Our result suggests further that multiple transcription factors are likely to function in these signal transduction cascades through common ABREs.

ABA-dependent stress responsive gene expression is critical to plant growth and productivity. Here, we reported a family of transcription factors that interact with *cis*-regulatory elements mediating this process. Although their specific roles *in planta* remains to be determined, our data presented here suggest that they are likely to be involved in various ABA-mediated stress responses. They can bind to ABREs highly conserved among stress responsive promoters. They can transactivate an ABRE-containing reporter gene. Their expression is induced by ABA and by various environmental stresses. Hence, ABFs are excellent targets of genetic manipulation for the generation of stress tolerant transgenic plants.

### REFERENCES

Altschul, S.F., Gish, W., Miller, W., Myers, E.W. and Lipman, D.J. (1990). Basic local alignment search tool. *J. Mol. Biol*. 215, 403-410.
Ausubel, F.M., Brent,R., Kingston, R.E., Moore,D.D., Seidman, J.G., Smith, J.A. and Struhl, K. (1994). *Current Protocols in Molecular Biology* (New York: Green Publishing Associates/Wiley Interscience). de Bruxelles G.L., Peacock, W.J., Dennis, E.S. and Dolferus, R. (1996). Abscisic acid induces the alcoholdehydrogenase gene in *Arabidopsis*. *Plant Physiol*. 111, 381-391.
Busk, P.K., Jensen, A.B. and Pages, M. (1997). Regulatory elements *in vivo* in the promoter of the abscisic acid responsive gene *rab17* from maize. *Plant J*. 11, 1285-1295.
Busk, P.K. and Pages, M. (1998). Regulation of abscisic acid-induced transcription. *Plant Mol. Biol*. 37, 425-435.
Chomczynski, P. and Mackey, K. (1995). Modification of the TRI reagent procedure for isolation of RNA from polysaccharide- and proteoglycan-rich sources. *BioTechniques* 19, 942-945.
Foster, R., Izawa, T. and Chua, N.-H. (1994). Plant bZIP proteins gather at ACGT elements. FASEB J. 8, 192-199.
Guiltinan. M.J., Marcotte, W.R. and Quatrano, R.S. (1990). A plant leucine zipper protein that recognizes an abscisic acid responsive element. *Science* 250, 267-271.
Guthrie, C. and Fink, G.R. eds. (1991). Guide to Yeast Genetics and Molecular Biology. *Methods Enzymol* 194.
Hollung, K., Espelund, M., Schou, K., and Jakobsen, K.S. (1997) *Plant Mol. Biol*. **35**, 561-571.
Ingram, J. and Bartels, D. (1996). The molecular basis of dehydration tolerance in plants. *Ann. Rev. Plant Physiol. Plant Mol. Biol*. 47, 377-403.
Ishitani, M., Xiong, L., Stevenson, B. and Zhu, J.-K. (1997). Genetic analysis of osmotic and cold stress signal transduction in *Arabidopsis*: interactions and convergence of abscisic acid-dependent and abscisic acid-independent pathways. *Plant Cell* 9, 1935-1949.
Jaglo-Ottosen, K. R., Gilmour S.J., Zarka, D.G., Schabenberger and Thomashow, M.F. (1998). *Arabidopsis* CBF1 overexpression induces COR genes and enhances freezing tolerance. *Science* 280, 104-106.
Kasuga, M., Liu, Q., Miura, S., Yamaguchi-Shinozaki, K. and Shinozaki, K. (1999). Improving plant drought, salt, and freezing tolerance by gene transfer of a single stress-inducible transcription factor. *Nature Biotech*. 17, 287-291.
Kemp, B.E. and Pearson, R.B. (1990). Protein kinase recognition sequence motifs. *Trends Biochem. Sci*. 15, 342-346.
Kim, S.Y. and Thomas, T.L. (1998). A family of basic leucine zipper proteins bind to seed-specification elements in the carrot *Dc3* gene promoter. *J. Plant Physiol*. 152, 607-613.
Kim, S.Y., Chung, H.-J. and Thomas, T.L. (1997). Isolation of a novel class of bZIP transcription factors that interact with ABA-responsive and embryo-specification elements in the *Dc3* promoter using a modified yeast one-hybrid system. *Plant J*. 11, 1237-1251.
Kusano, T., Berberich, T., Harada, M., Suzuki, N. and Sugawara, K. (1995). A maize DNA-binding factor with a bZIP motif is induced by low temperature. *Mol. Gen.Gent.* 248, 507-517.
Lam, E. and Chua, N.-H. (1991). Tetramer of a 21-base pair synthetic element confers seed expression and transcriptional enhancement in response to water stress and abscisic acid. J. Biol. Chem. 266, 17131-17135.
Landschulz, W.H., Johnson, P.F. and McKnight, S.L. (1988). The leucine zipper: A hypothetical structure common to a new class of DNA binding proteins. *Science* 240, 1759-1764.
Leung, J. and Giraudat, J. (1998). Abscisic acid signal transduction. *Ann. Rev. Plant physiol. Plant Mol. Biol*. 49, 199-222.
Liu, Q, Kasuga, M., Sakuma, Y., Abe, H., Miura, S., Yamaguchi-Shinozaki, K. and Shinozaki, K. (1998). Two transcription factors, DREB1 and DREB2, with an EREBP/AP2 DNA binding domain separate two cellular signal transduction pathways in drought- and low temperature-responsive gene expression, respectively, in *Arabidopsis*. *Plant Cell* 10, 1391-1406.
Lu, G., Paul, A.-L., McCarty D.R. and Ferl, R.J. (1996). Transcription factor veracity: Is GBF3 responsible for ABA-regulated expression of Arabidopsis *Adh*? *Plant Cell* 8, 847-857.
Ma, J. and Ptashne, M. (1987). A new class of yeast transcriptional activators. *Cell* 51, 113-119.
Menkens, A.E., Schindler U. and Cashmore, A.R. (1995). The G-box: a ubiquitous regulatory DNA element in plants bound by the GBF family of bZIP proteins. *Trend Biochem Sci*. 20, 506-510.
Nakagawa, H., Ohmiya, K. and Hattori, T. (1996). A rice bZIP protein, designated as *OSBZ8*, is rapidly induced by abscisic acid. *Plant J*. 9, 217-227.
Oeda, K., Salinas, J. and Chua, N.-H. (1991). A tobacco bZIP transcription activator (TAF-1) binds to a G-box-like motif conserved in plant genes. *EMBO J.* 10, 1793-1802.
Ono, A., Izawa, T., Chua, N.-H. and Shimamoto, K. (1996). The rice rab16B promoter of rice contains two distinct abscisic acid-responsive elements. *Plant physiol*. 112, 483-491.
Pla, M., Vilardell, J., Guiltinan, M.J., Marcotte, W.R., Niogret, M.F., Quatrano, R.S. and Pages, M. (1993). The *cis*-regulatory element CCACGTGG is involved in ABA and water-stress response of the maize gene *rab28*. *Plant Mol. Biol*. 21, 259-266.
   Pollock, R. and Treisman. R. (1990). A sensitive method for the determination of protein-DNA binding specificities. *Nucl Acids Res*. 18, 6197-6204.
Sambrook, J., Fritsch, E.F. and Maniatis, T. (1989). *Molecular Cloning: A laboratory Manual*, 2^{nd} ed. (Cold Spring Harbor, NY: Cold Spring Harbor Laboratory Press).
Schindler, U., Menkens, A.E., Beckmann, H., Ecker, J.R. and Cashmore, A.R. (1992). Heterodimerization between light-regulated and ubiquitously expressed *Arabidopsis* GBF bZIP proteins. *EMBO J*. 4, 1261-1273.
Shinozaki, K. and Yamaguchi-Shinozaki, K. (1996). Molecular responses to drought and cold stress. *Curr. Opin. Biotech*. 7,161-167.
Thomas, T.L., Chung, H.-J. and Nunberg, A.N. (1997). ABA signaling in plant development and growth. In *Signal Transduction in Plants*, (Aducci, P. ed.). Basel: Birkhauser Verlag, pp. 23-43.
Thomashow, M.F. (1998). Role of cold-responsive genes in plant freezing tolerance. *Plant Physiol*. 118, 1-7.
Thompson, J.D., Higgins, D.G. and Gibson, T.J. (1994). CLUSTAL W: improving the sensitivity of progressive multiple sequence alignment through sequence weighting, position-specific gap penalties and weight matrix choice. *Nucleic Acids Res*. 22, 4673-4680.
Yamaguchi-Shinozaki, K., and Shinozaki, K. (1994). A novel *cis*-acting element in an *Arabidopsis* gene is involved responsiveness to drought, low-temperature, or high-salt stress. *Plant Cell* 6, 251-264.

## Claims

1. An isolated nucleic acid molecule encoding the Abscisic acid responsive element binding factor 3(ABF3) having amino acid sequence of SEQ ID No. 6.

2. The isolated nucleic acid molecule of claim 1, wherein the nucleic acid molecule is a messenger RNA molecule.

3. The isolated nucleic acid molecule of claim 1, wherein the nucleic acid molecule is a cDNA molecule having the nucleotide sequence of SEQ ID No. 5.

## Patentansprüche

1. Isoliertes Nukleinsäuremolekül, welches den Bindungsfaktor 3 für das auf Abscisinsäure ansprechende Element (ABF3) mit der Aminosäuresequenz von SEQ ID NO:6 codiert.

2. Isoliertes Nukleinsäuremolekül nach Anspruch 1, wobei das Nukleinsäuremolekül ein Boten-RNA-Molekül ist.

3. Isoliertes Nukleinsäuremolekül nach Anspruch 1, wobei das Nukleinsäuremolekül ein cDNA-Molekül mit der Nukleotidsequenz von SEQ ID NO:5 ist.

## Revendications

1. Molécule d'acide nucléique isolée codant pour le facteur 3 de liaison à l'élément sensible à l'acide abscisique (ABF3) ayant la séquence d'aminoacides de la SEQ ID N°6.

2. Molécule d'acide nucléique isolée suivant la revendication 1, ladite molécule d'acide nucléique étant une molécule d'ARN messager.

3. Molécule d'acide nucléique isolée suivant la revendication 1, ladite molécule d'acide nucléique étant une molécule d'ADNc ayant la séquence de nucléotides de la SEQ ID N°5.
